# EUROPÄISCHE PATENTSCHRIFT

(11) **EP 3 469 350 B1**
(45) Veröffentlichungstag und Bekanntmachung des Hinweises auf die Patenterteilung: **05.07.2023**
(21) Anmeldenummer: 17716199.9
(22) Anmeldetag: 06.04.2017
(51) Int. Cl.: G01N 29/032, G01N 29/22, G01N 29/24, G01N 29/44, G01N 15/02, G01N 15/06, G01N 29/48

(54) **VERFAHREN, VORRICHTUNG UND VERWENDUNG DER VORRICHTUNG ZUR QUANTITATIVEN BESTIMMUNG DER KONZENTRATION ODER PARTIKELGRÖSSEN EINER KOMPONENTE EINES HETEROGENEN STOFFGEMISCHES**
METHOD, DEVICE AND USE FOR THE DEVICE FOR QUANTITIVELY DETERMINING THE CONCENTRATION OR PARTICLE SIZE OF A COMPONENT OF A HETEROGENEOUS MATERIAL MIXTURE
PROCÉDÉ, DISPOSITIF ET UTILISATION DUDIT DISPOSITIF POUR LA DÉTERMINATION QUANTITATIVE DE LA CONCENTRATION OU DE LA TAILLE DES PARTICULES D'UN COMPOSANT D'UN MÉLANGE HÉTÉROGÈNE DE SUBSTANCES

(30) Priorität: 14.06.2016 DE 102016007173
(43) Veröffentlichungstag der Anmeldung: 17.04.2019
(73) Patentinhaber: Fraunhofer-ges. zur Förderung der Angewandten Forschung E.V., 80686 München (DE); Nemak, S.A.B. de C.V., 66000 García, Nuevo León (MX); Hydro Aluminium Rolled Products GmbH, 41515 Grevenbroich (DE); Inoson GmbH, 66386 St. Ingbert (DE)
(72) Erfinder: WASCHKIES, Thomas, 66440 Blieskastel (DE); REUTHER, Andrea, 66787 Wadgassen (DE); LICHT, Rudolf, 66440 Blieskastel (DE); WEIKERT-MÜLLER, Miriam, 66123 Saarbrücken (DE); FEIKUS, Friederike, 52072 Aachen (DE); FISCHER, Sebastian, 60549 Frankfurt (DE); BADOWSKI, Mark, 53117 Bonn (DE); HAHN-JOSÉ, Thomas, 66386 St. Ingbert (DE)
(74) Vertreter: Rösler, Uwe
(86) Internationale Anmeldenummer: PCT/EP2017/058242
(87) Internationale Veröffentlichungsnummer: WO 2017/215807

(56) Entgegenhaltungen:
- WO-A1-2012/004114
- DE-T2- 60 001 951
- US-A- 4 287 755
- J W Griffin ET AL: "Under-Sodium Viewing: A Review of Ultrasonic Imaging Technology for Liquid Metal Fast Reactors", , 1. März 2009 (2009-03-01), XP055372597, Washington, United States Gefunden im Internet: URL:www.pnl.gov/publications [gefunden am 2017-05-15]
- ONO Y ET AL: "AN ON-LINE ULTRASONIC CLEANLINESS ANALYZER FOR MOLTEN LIGHT METALS", JOM: JOURNAL OF METALS, SPRINGER NEW YORK LLC, UNITED STATES, vol. 56, no. 2, 1 February 2004 (2004-02-01), pages 59-64, XP001244015, ISSN: 1047-4838, DOI: 10.1007/S11837-004-0148-9

## Beschreibung

### Technisches Gebiet

Die Erfindung betrifft ein Verfahren sowie eine Vorrichtung zur quantitativen Bestimmung von Anzahl und Größe von in einem Gefäß fließenden Medium enthaltenen partikulären Komponenten, bei dem Ultraschallwellen in das fließende Medium einkoppeln, die zumindest teilweise an den partikulären Komponenten reflektieren und deren reflektierte Ultraschallwellenanteile in Form von Ultraschallzeitsignalen detektiert werden, die der quantitativen Bestimmung zugrunde gelegt werden.

### Stand der Technik

Unter anderem in der metallverarbeitenden und chemischen Industrie besteht häufig die Aufgabe insbesondere heterogene Stoffgemische wie Suspensionen quantitativ zu qualifizieren. Beispiele sind Metallschmelzen, die neben dem Metall oder Mischungen von Metallen Verunreinigungen oder erwünschte bzw. unerwünschte weitere Komponenten enthalten, zum Beispiel Partikel in Metallschmelzen bestehend aus Oxiden, Chloriden, Karbiden, Nitriden, Boriden und/oder intermetallischen Phasen. In der chemischen Industrie treten beispielsweise bei der Herstellung von Polymeren während der Polymerisation heterogene Stoffgemische auf. In beiden Beispielen ist es wünschenswert genaue quantitative Aussagen zu den Komponenten eines Stoffgemisches zu treffen, d.h. Partikelanzahl, Partikelkonzentration und/oder Partikelgrößen zu bestimmen, um Produktionsprozesse zu steuern, zu regeln oder zu überwachen.

Im Bereich des ultraschallbasierten Partikelnachweises in beispielsweise Aluminiumschmelze ist das sogenannte MetalVision-Verfahren aus Kurban M., Sommerville I.D., Mountford N.D.G., Montford P.H., An ultrasonic sensor for the Continuous Online Monitoring of the Cleanliness of Liquid Aluminium, Light Metals 2005, TMS, 945-949 bekannt, welches im Bereich des Aluminium-Strangguss verwendet wird. Bei diesem Verfahren wird Ultraschall über parallel angeordnete Stahlwellenleiter in die flüssige Aluminiumschmelze eingekoppelt. Nachteilig ist die mangelnde Reproduzierbarkeit der Messergebnisse.

Aus den Druckschriften EP 1 194 772 B1 bzw. DE 600 01 951 T2 sind ein Verfahren sowie eine Vorrichtung zur individuellen Visualisierung, Größenmessung und Zählung von suspendierten Einschlüssen in einer in einem Gefäß befindlichen Metallschmelze mittels Ultraschall zu entnehmen.

Mit wenigstens einem Ultraschallwandler werden Ultraschallwellen in Form einzelner sogenannter Ultraschallschüsse erzeugt und vermittels eines Wellenleiters in die zu untersuchende Metallschmelze eingekoppelt, in der sie an in der Metallschmelze enthaltenden Einschlüssen teilweise reflektiert werden. Die reflektierten Ultraschallwellen werden mittels eines Ultraschallwellendetektors detektiert und zu Zwecken ihrer Zählung und Vermessung sowie Visualisierung mittels einer Bildanalyse ausgewertet. Der Bildanalyse sowie der quantitativen Vermessung der aus den detektierten Ultraschallwellen erhaltenen Echosignalen wird eine Eichkurve zugrunde gelegt, die im Rahmen eines Eichschrittes gewonnen wurde, bei dem wenigstens ein Eichreflektor bekannter stabiler Größe zum Einsatz kommt. Der Eichreflektor wird hierzu in die Metallschmelze im Bereich des sogenannten Brennfleckes positioniert, in dem die Ultraschallwellen mit der Metallschmelze wechselwirken und aus dem reflektierte Ultraschallwellenanteile austreten und von wenigstens einem Ultraschalldetektor erfassbar sind. Die Eichkurve stellt einen funktionellen Zusammenhang zwischen den Amplituden detektierter Echosignale und den Durchmessern der Hindernisse, an denen die Echosignale reflektiert worden sind, her.

Die Druckschrift US 2013/104657 offenbart ein Ultraschall-Partikel-Messsystem, mit dem Ultraschallwellen über eine akustische Linse in eine ein Messrohr durchströmende, Partikel enthaltende Flüssigkeit, zum Beispiel wässrige Lösung eingekoppelt werden. Mit einer Auswerteeinheit werden die Beträge der Amplituden der vom Ultraschallwandler empfangenen Reflexionssignale sowie die Anzahl der Amplituden in einem vorgegebenen zeitlichen Intervall gezählt, die größer als ein vorgegebener Schwellwert sind. Insbesondere werden die Ultraschallsignale aus einem bestimmten Volumenelement ausgewertet.

Die Druckschrift US 4 287 755 A2 offenbart eine Sonde mit einer vom geschmolzenen Aluminium durchströmbaren Öffnung, die über eine Fläche Ultraschallwellen in die Aluminiumschmelze einkoppelt. Ein Teil der Ultraschallwellen wird an einer Reflektorfläche reflektiert. Die Reflexionssignale von der Reflektorfläche dienen lediglich der Detektion und Evaluation von der Fließgeschwindigkeit der Schmelze, der Amplitudendämpfung sowie einer Frequenzverschiebung der Ultraschallsignale. Für eine verbesserte Benetzung der Sonde wird zudem vorgeschlagen, eine Titan-Sonde mit Aluminium zu beschichten.

Die Druckschrift ONO Y ET AL: "AN ON-LINE ULTRASONIC CLEANLINESS ANALYZER FOR MOLTEN LIGHT METALS",JOM: JOURNAL OF METALS, SPRINGER NEW YORK LLC, UNITED STATES, Bd. 56, Nr. 2, 1. Februar 2004 (2004-02-01), Seiten 59-64 offenbart ein Ultraschall-Messsystem zur Partikelmessung in einer Metallschmelze mittels akustischer Wellenleiter.

### Darstellung der Erfindung

Der Erfindung liegt die Aufgabe zugrunde, ein Verfahren und eine Vorrichtung sowie deren Verwendung bereitzustellen, mit dem Ziel eine Partikelanzahl, Partikelkonzentration und/oder Partikelgrößen in Stoffgemischen, Flüssigkeiten, Suspensionen insbesondere Schmelzen mit hoher Genauigkeit und Reproduzierbarkeit zu bestimmen. Ferner gilt es den verfahrens- und vorrichtungstechnischen Aufwand zu reduzieren.

Die gestellte technische Aufgabe wird durch ein Verfahren nach Anspruch 1, eine Vorrichtung nach Anspruch 8 und eine Verwendung der Vorrichtung nach Anspruch 10 gelöst. Weitere vorteilhafte Ausführungsformen ergeben sich aus den zusätzlichen Merkmalen der abhängigen Ansprüche.

Das lösungsgemäße Verfahren gemäß den Merkmalen des Oberbegriffes des Anspruches 1 erlaubt eine unmittelbare Einschallung bzw. Einkopplung der Ultraschallwellen in das fließende Medium, so dass zumindest ein Teil der eingekoppelten Ultraschallwellen einer Reflexion an einem das fließende Medium begrenzenden Wandbereich des Gefäßes oder an einem innerhalb des Gefäßes eingebrachten Reflektors unterliegen, durch die ein dem Wandbereich oder dem Reflektor zuordenbares Echo-Ultraschallzeitsignal erzeugt wird. Die Einkopplung der Ultraschallwellen erfolgt in eine Metallschmelze, innerhalb der es gilt partikuläre Komponenten quantitativ zu erfassen. Auch ist es möglich beliebige heterogene flüssige Stoffgemische, wie Suspensionen, mittels Ultraschallwellen-Einkopplung zu Zwecken einer quantifizierenden Bestimmung von partikulären Komponenten zu untersuchen.

Dies liegt jedoch nicht innerhalb des Schutzumfangs der Ansprüche.

Das Einkoppeln der Ultraschallwellen in das fließende Medium erfolgt mit einer quer zur Strömungsrichtung des fließenden Mediums orientierten Hauptausbreitungsrichtung, wobei die sich längs der Hauptausbreitungsrichtung ausbreitenden Ultraschallwellen vorzugsweise orthogonal auf das Gefäß begrenzenden Wandbereich auftreffen und an diesem reflektiert werden. Die Detektion der längs der gesamten Ultraschalllaufstrecke innerhalb des fließenden Mediums reflektierten Ultraschallwellenanteile einschließlich der an dem in Hauptausbreitungsrichtung rückwärtig begrenzenden Wandbereich reflektierten Ultraschallwellen erfolgt am Bereich oder exakt am Ort der Ultraschallwelleneinkopplung. Auf diese Weise werden Ultraschallzeitsignale längs des gesamten Weges zwischen Einkoppelort und rückwärtiger Begrenzungswand erhalten. Die an der Begrenzungswand hervorgerufene Ultraschallwellenreflexion zeichnet sich als charakteristisches Echo-Ultraschallzeitsignal aus, das für die Ermittlung wenigstens einer Amplitudenschwellwertfunktion verwendet wird, die zu jedem detektierten Ultraschallzeitsignal einen Amplitudenschwellwert festlegt.

Bei Einsatz bspw. von zwei oder mehr Wellenleiter, von denen wenigstens ein zweiter Wellenleiter als Empfänger dient, kann der Ort der Detektion vom Ort der Einkopplung abweichen. Dies liegt jedoch nicht innerhalb des Schutzumfangs der Ansprüche.

Der Einsatz eines zusätzlich in das Gefäß eingebrachten Reflektors, der über eine glatte, vorzugsweise ebene Reflektoroberfläche verfügt, wird in jenen Fällen vorgenommen, in denen der Abstand zwischen der Ultraschallwelleneinkopplung und dem das Gefäß begrenzenden Wandbereich zu groß ist und/oder der Wandbereich, bspw. durch Ablagerungen, ungeeignet ist Ultraschallwellen möglichst verlustarm zu reflektieren.

### Vorzugsweise wird zur Ermittlung der wenigstens einen

Amplitudenschwellwertfunktion wenigstens eine der nachfolgenden physikalischen Eigenschaften berücksichtigt:
a) die Ultraschallfeldverteilung innerhalb des fließenden Medium, d.h. die räumliche Ausdehnung und Intensität der sich richtungsabhängig innerhalb des fließenden Mediums ausbereitenden Ultraschallwellen, bspw. in Form von Haupt- und Nebenkeulen,
b) die akustische Dämpfung der Ultraschallwellen im fließenden Medium, d.h. die medium-spezifische und Medium bedingte Abnahme der Ultraschallwellenamplituden mit fortschreitender Ausbreitung innerhalb des fließenden Mediums,
c) die Einkoppelbedingungen der Ultraschallwellen in das fließende Medium, d.h. die Güte, mit der die initiale Ultraschallwellenenergie, mit der Ultraschallwellen generiert werden, bspw. mittels eines piezoelektrischen Ultraschallwandlers, im Rahmen der sich innerhalb des fließenden Mediums ausbreitenden Ultraschallwellen transformiert wird. So äußern sich verändernde Einkoppelbedingungen unmittelbar in einem sich verändernden Echo-Ultraschallzeitsignal, dessen unmittelbarer Einfluss auf die Amplitudenschwellwertfunktion Auswirkung auf die Amplitudenschwellwerte sämtlicher Ultraschallzeitsignale besitzt. In diesem Fall werden die Amplitudenschwellwertfunktionen für alle zeitlich folgenden Ultraschallzeitsignale korrigiert. Die zeitlich zurückliegenden Amplitudenschwellwerte müssen jedoch nicht korrigiert werden.

Zu Zwecken der quantitativen Erfassung und Auswertung von in dem fließenden Medium enthaltenen partikulären Komponenten werden in einem weiteren Schritt sämtliche den einzelnen erfassten Ultraschallzeitsignalen zugeordnete Amplitudenwerte erfasst, die jeweils größer sind als ein zu den jeweiligen Ultraschallzeitsignalen festgelegter Amplitudenschwellwert.

In vorteilhafter Weise werden nicht alle Ultraschallzeitsignale, die von Reflexionsereignissen innerhalb des fließenden Mediums zwischen dem Ort der Einkopplung und an dem rückwärtigen Wandbereich herrühren, zur weiteren Auswertung herangezogen. Vielmehr wird ein Auswertezeitbereich festgelegt, der einem räumlichen Messbereich innerhalb des fließenden Mediums längs der Hauptausbreitungsrichtung entspricht und der beliebig zwischen dem Ort des Einkoppelns und dem das Gefäß rückwärtig begrenzenden Wandbereich liegt. Je nach Untersuchungsvorgaben können der Auswertezeitbereich und das damit verbundene, auswertbare Messvolumen geeignet dimensioniert werden.

Die für die Auswertung der Ultraschallzeitsignale innerhalb eines vorgebbaren Auswertezeitbereiches erforderliche Amplitudenschwellwertfunktion ist im einfachsten Fall eine horizontale Gerade, die in geeigneter Weise zum numerischen Vergleich mit den detektierten Ultraschallzeitsignalen überlagert wird.

Je nach Anforderungen an die weitere Auswertung der Ultraschallzeitsignale können die vorstehend genannten Aspekte, wie Ultraschallfeldverteilung, Dämpfung, Einkoppelbedingungen o.ä. in der Amplitudenschwellwertfunktion entsprechend berücksichtigt werden.

Gleichfalls kann der Verlauf der Amplitudenschwellwertfunktion einem logarithmischen oder exponentiellen Verlauf folgen. Die akustische Dämpfung des fließenden Mediums folgt bspw. einer Exponentialfunktion mit negativem Exponenten. Durch Multiplikation der Amplitudenschwellwertfunktion mit einer Exponentialfunktion mit positivem Exponenten, lässt sich auf diese Weise der Dämpfungseinfluss korrigieren.

Alternativ oder in Kombination mit der vorstehenden Dämpfungskorrektur kann der Verlauf der Amplitudenschwellwertfunktion linear mit positiver oder negativer Steigung gewählt sein. Z.B. nimmt der Schalldruck ausgehend von einem ebenen, kreisförmigen Schwinger mit zunehmenden Abstand z zum Ort der Einkopplung der Ultraschallwellen in ein Medium, d.h. im sogenannten Fernfeld, näherungsweise mit 1/z ab. Durch Multiplikation der Amplitudenschwellwertfunktion mit einer Funktion mit positiver Steigung kann demnach dieser Einfluss korrigiert werden.

Auch ist es möglich die Einkopplung der Ultraschallwellen in das fließende Medium fokussiert vorzunehmen, d.h. die Ultraschallwellen werden in einen längs der Hauptausbreitungsrichtung befindlichen Fokuspunkt fokussiert, der in Hauptausbreitungsrichtung stets vor dem das Gefäß begrenzenden Wandbereich liegt.

Die Lage des Fokuspunktes relativ zum Auswertezeitbereich, bzw. zum festgelegten Messvolumen, kann grundsätzlich beliebig, d.h. entweder innerhalb oder außerhalb des Auswertezeitbereiches gewählt werden.

Liegt der Ultraschallfokus hingegen innerhalb des Auswertezeitbereichs ist es vorteilhaft, am Fokuspunkt den niedrigsten Amplitudenschwellwert zu definieren, der beidseitig mit zunehmendem Abstand zum Fokuspunkt zunimmt. Liegt hingegen der Ultraschallfokus außerhalb des Auswertezeitbereichs ist es vorteilehaft, dass die Amplitudenschwellfunktion eine positive oder negative Steigung besitzt.

Grundsätzlich kann der Verlauf der Amplitudenschwellwertfunktion durch Berücksichtigung mehrere Einflussgrößen einen sehr komplexen Funktionsverlauf annehmen. Auch bietet es sich an mehrere unterschiedliche Amplitudenschwellwertfunktionen anzuwenden, um bspw. Partikelgrößenverteilungen entsprechend ermitteln zu können. Eine praktikable Anzahl unterschiedlicher Amplitudenschwellwertfunktionen liegt in der Regel im Bereich von 1 bis 10.

Nach Erfassung aller innerhalb des Auswertezeitbereiches jeweils betragsmäßig über der Amplitudenschwellwertfunktion liegenden Amplitudenwerten gilt es schließlich diesen Amplitudenwerten Werte zuzuordnen, die die Größe und/oder Anzahl der erfassten partikulären Komponenten innerhalb des fließenden Mediums beschreiben.

So lässt sich die Anzahl der erfassten partikulären Komponenten auf der Grundlage der Anzahl oder statistischen Häufigkeit ermitteln, mit der die pro Ultraschallzeitsignal erfassten Amplitudenwerte über einem jeweils durch die Amplitudenschwellwertfunktion für ein Ultraschallzeitsignal festgelegten Amplitudenschwellwert liegen.

Hingegen basiert die Größeninformation über die partikulären Komponenten auf dem numerischen Betrag des Amplitudenwertes des Ultraschallzeitsignals, d.h. die Peakhöhe bzw. Amplitudengröße eines Ultraschallzeitsignals beschreibt die jeweilige Partikelgröße. Zu beachten ist dabei, dass die Peakhöhen der Ultraschallzeitsignale ebenfalls von den Ultraschalleinkoppelbedingungen abhängen, ein Umstand den es durch eine dynamische Anpassung der Amplitudenschwellwertfunktion zu berücksichtigen gilt.

Auf Basis der so gewonnenen Erkenntnisse können Partikelanzahl und relative Partikelgrößenverteilung der in dem fließenden Medium erfassten partikulären Komponenten bestimmt werden.

Gilt es die Partikelgrößen exakt in absoluten Werten anzugeben, so kann auf Referenztabellen, so genannten Lookup-Tabellen, zurückgegriffen werden. Gleichfalls ist es möglich Kalibrierungswerte oder Kalibrierungsfunktionen in getrennten Versuchsreihen zu ermitteln, indem Ultraschallzeitsignale erfasst werden, insbesondere deren Amplituden oder/und Signalformen, die durch Reflexion der Ultraschallwellen an einem bekannten Ultraschallreflektor gewonnen werden. Die so erhaltenen Kalibrierungswerte oder Kalibrierungsfunktionen können nachfolgend zur Ermittlung der wenigstens einen Amplitudenschwellwertfunktion zugrunde gelegt werden.

Ferner zeichnet sich eine Vorrichtung zur quantitativen Bestimmung von Anzahl und Größe von in einem Gefäß fließenden Medium enthaltenen partikulären Komponenten, gemäß den Merkmalen des Oberbegriffes des Anspruches 1 derart aus, dass zum Zwecke der Ultraschallwelleneinkopplung in das fließende Medium wenigstens ein mit einem Ultraschallwandler akustisch gekoppelter Wellenleiter zumindest abschnittsweise in das fließende Medium eintaucht, wobei der aus einem Wellenleitermaterial bestehende Wellenleiter wenigstens im Bereich, in dem der Wellenleiter in das fließende Medium eintaucht, mit einer äußeren Schicht umgeben ist, so dass die äußere Schicht zwischen dem sonstigen Wellenleitermaterial und dem fließenden Medium angeordnet ist, und
die äußere Schicht eine vom sonstigen Wellenleitermaterial abweichende Stoffzusammensetzung besitzt.

Der Wellenleiter weist vorzugsweise ein einseitig stumpfes, spitzzulaufendes oder geometrisch bestimmt geformtes, zur Einkopplung von fokussierten Ultraschallwellen in das fließende Medium geeignetes Wellenleiterende auf.

Dabei ist das Wellenleiterende zumindest abschnittweise von der äußeren Schicht umgeben, deren Stoffzusammensetzung in Abhängigkeit des fließenden Mediums derart gewählt ist, so dass sich die Stoffzusammensetzung in Kontakt mit dem fließenden Medium auflöst.

Die Stoffzusammensetzung der äußeren Schicht enthält wenigstens eine die Benetzung des fließenden Mediums an das Wellenleitermaterial hervorrufende und/oder unterstützende Substanz, die nicht mit dem fließenden Medium identisch ist. Die wenigstens eine Substanz ist ein Schmelzsalz.

Um das Schmelzsalz im Bereich des Wellenleiterendes zumindest für den Einsatz in einer Metallschmelze robust anzubringen, wird es von einem im Messmedium schmelzenden bzw. sich lösendem Material umgeben, bspw. mittels einer Aluminiumfolie. Das am Wellenleiterende angebrachte Schmelzsalz verdrängt mögliche Oxide an der Wellenleiteroberfläche und ermöglicht so den direkten Kontakt des Wellenleiters mit dem fließenden Medium, vorzugsweise in Form einer Aluminiumschmelze.

Weitere Einzelheiten sind der weiteren Beschreibung unter Bezugnahme auf die folgenden Ausführungsbeispiele zu entnehmen.

### Kurze Beschreibung der Erfindung

Es zeigen:
- Fig. 1: Vorrichtung zur Messung partikulärer Komponenten in einem fließenden Medium mittels eines Wellenleiters zur Einkopplung von Ultraschallwellen in das Medium,
- Fig. 2: Vorrichtung gemäß Figur 1 mit zwei Wellenleiter zur Einkopplung von Ultraschallwellen in das Medium,
- Fig. 3: Vorrichtung gemäß Figur 2 mit zwei Wellenleiter zur Einkopplung von fokussierten Ultraschallwellen in das Medium,
- Fig. 4: Diagrammdarstellung von Ultraschallzeitsignalen mit überlagerter Amplitudenschwellwertfunktion und Auswertezeitbereich und
- Fig. 5: Vorrichtung gemäß Figur 1 mit einem Wellenleiter mit einem Benetzungsschuh am Wellenleiterende.

### Wege zur Ausführung der Erfindung, gewerbliche Verwendbarkeit

Unter Bezugnahme auf die im Weiteren beschriebenen Figuren wird eine Vorrichtung beschrieben, die es erlaubt die Konzentration bzw. Anzahl sowie die Größe von partikulären Komponenten in einem fließenden Medium 3 bspw. in Form von Stoffgemischen, Schmelzen, Metallschmelzen, wie Aluminiumschmelzen, oder Flüssigkeiten mit großer Genauigkeit zu messen.

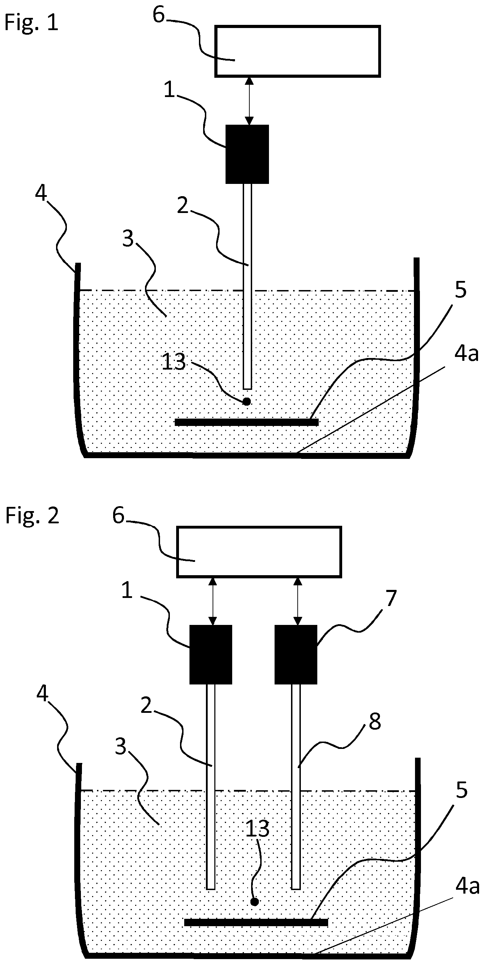

Wie in Fig. 1 dargestellt, wird Ultraschall von einem Ultraschallwandler 1, der als Sender dient erzeugt und über einen Wellenleiter 2, einem Mittel zum Einkoppeln des Ultraschalls, in die zu untersuchende Flüssigkeit eingekoppelt. Es sei angenommen, dass das fließende Medium 3 das Gefäß 4 orthogonal zur Zeichenebene durchströmt. Wie in Fig. 1 dargestellt, kann der gleiche Ultraschallwandler 1 auch als Empfänger des Ultraschallfeldes aus dem fließenden Medium 3 dienen.

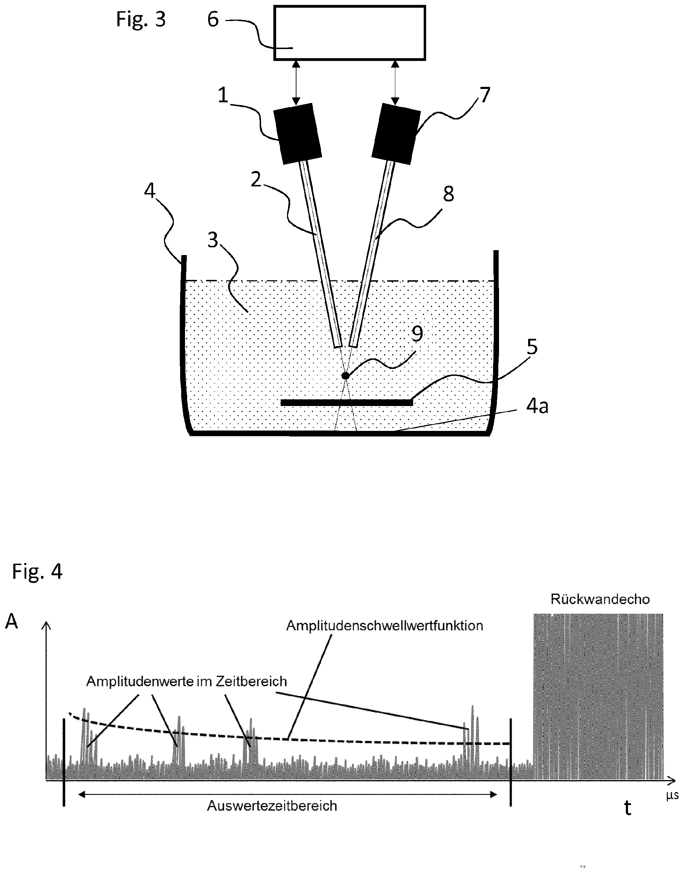

In Fig. 2 und Fig. 3 wird jeweils ein weiterer Ultraschallwandler 7 mit einem weiteren akustisch gekoppelten Wellenleiter 8 eingesetzt, wobei der Wellenleiter 8 den weiteren Ultraschallwandler 7 akustisch mit dem fließenden Medium 3 koppelt. Bei mehr als einem Ultraschallwandler sind diverse Betriebsmodi nutzbar, beispielsweise kann der Ultraschallwandler 1 als Sender und der Ultraschallwandler 7 als Empfänger dienen oder umgekehrt oder, beide Ultraschallwandler 1, 7 dienen als Sender und zeitversetzt als Empfänger. Die von mindestens einem Ultraschallwandler 1,7 empfangenen Ultraschallsignale werden durch die Messeinrichtung/ Auswertetechnik/ Auswerteeinrichtung 6 aufgezeichnet und ausgewertet. Die Auswerteeinrichtung 6 erfasst die Reflexionen bzw. Echos des Ultraschallfeldes aus dem fließenden Medium 3 sowie die Reflexion von einem optional in das fließende Medium 3 eingebrachten Ultraschallreflektor 5. Vorzugsweise dient ein in Ausbreitungsrichtung der Ultraschallwellen begrenzender Wandbereich 4a des Gefäßes 4, welches das Stoffgemisch mindestens teilweise umschließt, als Ultraschallreflektor 5. Das Echo des Ultraschallreflektors 5, 4a dient zur Kalibrierung der Auswertung der Echos aus dem fließenden Medium 3. Zur Auswertung der Echos aus dem fließenden Medium 3 kommt mindestens eine Amplitudenschwellwertfunktion zur Anwendung, die auf Basis des Echos des Ultraschallreflektors 5, 4a festgelegt wird.

Eine bevorzugte Vorrichtung besteht unter anderem aus dem mindestens einen Ultraschallwandler 1, den an den Ultraschallwandler 1 akustisch gekoppelten Wellenleiter 2, einem Behälter 4 zur Aufnahme bzw. zum Durchströmen mit einer fließenden Medium 3, insbesondere Suspension, einem in dem Medium angeordneten Ultraschallreflektor 5 oder 4a, wobei der Wellenleiter 2 so in das Medium 3 hineinragt und so zum Ultraschallreflektor 5, 4a angeordnet ist, dass ein sich in dem Medium 3 ausbildendes Ultraschallfeld mindestens einen Fokus 13 aufweist, der räumlich zwischen dem Ultraschallreflektor 5, 4a, und dem ersten Wellenleiter 2 und/oder einem weiteren Wellenleiter 8 liegt.

Eine weitere bevorzugte Vorrichtung besteht unter anderem aus mindestens einer mit dem mindestens einen Ultraschallwandler 1 gekoppelten Auswerteeinrichtung 6, den an den Ultraschallwandler 1 akustisch gekoppelten Wellenleiter 2, einem Behälter 4, das von einem fließenden Medium 3, insbesondere in Form einer Suspension, durchströmt wird, wobei der Wellenleiter 2 in das Medium 3 hineinragt und eingerichtet ist, ein vom Ultraschallwandler 1 erzeugtes Ultraschallfeld in das Medium 3 einzukoppeln und Reflexionen des Ultraschallfeldes an Grenzflächen im fließenden Medium 3 insbesondere an Partikeln in dem Medium 3, in den Ultraschallwandler als Ultraschallzeitsignale einzukoppeln, wobei die Auswerteeinrichtung eingerichtet ist unter Nutzung einer Schwellwertfunktion Energiemaxima oder/und Leistungsmaxima im Empfangszeitsignal zu erfassen und zu zählen.

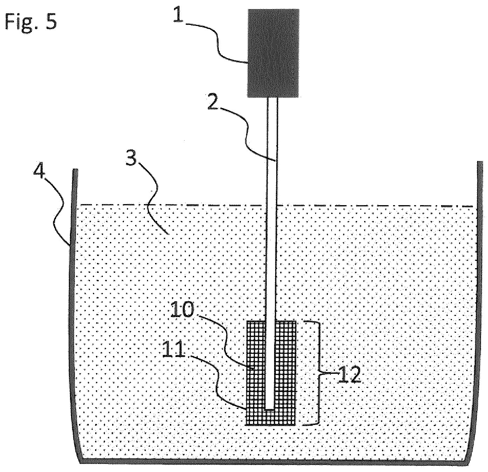

Eine weitere bevorzugte Vorrichtung besteht mindestens aus dem an den mindestens einen Ultraschallwandler 1 akustisch gekoppelten Wellenleiter 2, der in ein zu untersuchendes fließendes Medium, insbesondere Suspension hineinragt, wobei der Wellenleiter 2 mindestens teilweise eine äußere Schicht 10 mit einer vom sonstigen Wellenleitermaterial abweichende Stoffzusammensetzung aufweist und die äußere Schicht 10 zwischen dem sonstigen Wellenleitermaterial und dem fließenden Medium 3 angeordnet ist, siehe Figur 5.

Als Messvolumen wird der Raum bezeichnet, der durch das Ende des mindestens einen Wellenleiters 2 und durch den Ultraschallreflektor 5, 4a definiert wird.

Ein Fokus 13 des Ultraschallfeldes befindet sich vorzugsweise im Messvolumen.

Das vom Ultraschallreflektor 5, 4a verursachte Echo wird auch Rückwandecho genannt, die Begriffe sind austauschbar.

Der im Aufbau integrierte Ultraschallreflektor 5 erzeugt im Ultraschallsignal ein Rückwandecho nach Fig. 4. Dieses dient als Kalibrierung des Ultraschallsignals, da hierin die eingekoppelte Ultraschallenergie repräsentiert wird. Die Kalibrierung bezieht sich auf eine Aussage zur absoluten Partikelgröße, indem sie in die Festlegung der Amplitudenschwellwertfunktion einfließt. Auch kann das Rückwandecho zur Funktionsprüfung des Messsystems verwendet werden, da Einkoppelschwankungen des Ultraschalls von Wellenleitern in die Flüssigkeit nachgewiesen und hierdurch auch korrigiert werden können.

Der Ultraschallreflektor wird in Abhängigkeit von den eingesetzten Wellenleitern innerhalb des Messmediums positioniert. Hierbei sind vorzugsweise die folgenden Anordnungen möglich:
a) Werden die Wellenleiter unter einem Winkel zueinander angeordnet (Fig. 3) ist die höchste Ultraschallamplitude durch den Fokus 9, welcher sich aus dem Schnittpunkt der gedachten Verlängerung der beiden Wellenleiter ergibt, gegeben. Der Ultraschallreflektor wird in einem Abstand von dem Wellenleiter so angeordnet, dass der Fokus 9 zwischen dem Wellenleiter auf der einen Seite und dem Utraschallreflektor auf der anderen Seite liegt. Der Abstand des Fokus 9 vom Ultraschallreflektor liegt vorzugsweise im Bereich von 5 mm bis 80 mm.
   Für den Fall, dass die Tiefe des Behälters 4 nur sehr klein ist, kann es vorkommen, dass der Fokus 9 weiter entfernt liegt als der Reflektor 5 bzw. die Behälterwand. Dieser Fall ist zwar nicht ideal, allerdings ist eine Messung trotzdem möglich
b) Sind die Wellenleiter 2, 8 parallel zueinander angeordnet (Fig. 2) oder wird mit einem einzelnen Wellenleiter 2 gearbeitet (Fig. 1), ist die Position des Ultraschallreflektors 5, 4a durch den Fokus 13 des Ultraschallfeldes gegeben. Dieser Fokus 13 ist abhängig von der Geometrie der Enden der Wellenleiter 2, 8, welche sich im fließenden Medium 3 befinden. Der Ultraschallreflektor 5 bzw. die Begrenzungswand 4a des Gefäßes wird vorzugsweise in einem Abstand größer oder gleich (13) angeordnet. Auch in den Fällen des Aufbaus nach Fig. 1 und/oder Fig. 2 liegt der Fokus 13 des Ultraschallfeldes in einem Raum der einerseits durch die Enden der Wellenleiter und anderseits durch den Ultraschallreflektor 5 bzw. der Begrenzungswand 4a des Gefäßes begrenzt ist.
   Für den Fall, dass die Tiefe des Behälters 4 nur sehr klein ist, kann es vorkommen, dass der Fokus 9 weiter entfernt liegt als der Reflektor 5 bzw. die Behälterwand. Dieser Fall ist zwar nicht ideal, allerdings ist eine Messung trotzdem möglich.
c) Je nach Größe bzw. Tiefe des Gefäßes 4 kann der Fokus 13 in Ultraschallausbreitungsrichtung auch hinter dem Reflektor 5 bzw. hinter der Begrenzungswand 4a liegen, so bspw. auch außerhalb des Gefäßes 4.

Zum Vermessen einer Aluminiumschmelze als fließendes Medium 3 kann beispielsweise ein Aufbau nach Fig. 3 gewählt werden. Der Fokus 9 entspricht etwa einem Abstand von 50 mm zu den Enden der Wellenleiter 2, 8. Der Winkel zwischen den Wellenleitern 2,8 entspricht hierbei 8° bis 30°. Der Ultraschallreflektor 5 bzw. die Begrenzungswand 4a des Gefäßes besteht aus einem Warmarbeitsstahl. Hier sind insbesondere auch keramische Werkstoffe bzw. alle hochschmelzenden, im fließenden Medium schlecht benetzenden Werkstoffe, gut geeignet wie beispielsweise SiAlON, Siliziumnitrid, Aluminiumoxid.

Die Wellenleiter 2,8 werden vorzugsweise so gewählt, dass eine ausreichende Benetzung zum fließenden Medium hergestellt wird. Die Aufbauten der Wellenleiter entsprechen beispielsweise denen nach Fig. 1, 2, 3, 5.

Für die Aluminiumschmelzen als Medium können Wellenleiter aus Titan (Grade 2) eingesetzt werden. Weitere geeignete Wellenleitermaterialien sind Siliziumnitrid, SiAlON, Stahl (Warmarbeitsstahl 1018 H13 (USA) oder X40 CrMoV 5-1 und lösungsgeglühter Stahl (1.4436)). Die Wellenleiter haben beispielsweise Längen von 600 mm, 500 mm, 400 mm oder 300 mm und Durchmesser von 8 mm, 9 mm. 10 mm, 11 mm, 12 mm, 13 mm oder 14 mm.

Die Frequenz des Ultraschallfeldes liegt vorzugsweise im Frequenzbereich 2 MHz bis 12 MHz. Beispielsweise für eine Aluminiumschmelze als Messmedium hat sich eine Ultraschallfrequenz von 6 MHz oder 10 MHz als geeignet erwiesen, wobei eine Ultraschallfrequenz von circa 10 MHz besonders bevorzugt ist.

Zur Bewertung der Partikelanzahl im fließenden Medium wird ein Auswertezeitbereich nach Fig. 4 vor dem Rückwandecho bzw. Ultraschallreflektorecho gewählt. Durch die Auswahl des Auswertezeitbereich lässt sich das Messvolumen individuell einstellen. Ein kleinerer Auswertezeitbereich entspricht einem kleineren Messvolumen.

Der Auswertezeitbereich ist hierbei stark an die Ultraschallfelder im Medium gekoppelt, da eine ausreichende Ultraschallenergie notwendig ist.

Für die Aluminiumschmelzen wird ein Auswertezeitbereich gewählt, der etwa 4 cm im Medium entspricht. Das Ende des Auswertezeitbereichs liegt knapp vor dem Rückwandecho (Fig. 4). Da durch diesen Zeitbereich das Messvolumen eingestellt werden kann ist prinzipiell auch ein deutlich kürzerer aber, bei ausreichender Ultraschallenergie, auch deutlich längerer Zeitbereich möglich.

Vorzugsweise wird die Anzahl an Amplitudenwerten innerhalb des gewählten Zeitbereichs gezählt, welche eine bestimmte Amplitudenschwellwertfunktion überschreiten (siehe Fig. 4). Der Zählwert ist proportional zur Partikelkonzentration, sodass mit Hilfe einer Kalibrierungsfunktion aus dem Zählwert eine konkrete Partikelkonzentration berechnet werden kann.

Für eine Aluminiumschmelze sind so die relevanten und vom Messsystem erfassbaren Konzentrationsbereiche im Bereich von 100 Partikel bis 100.000 Partikel pro kg Aluminiumschmelze erfassbar.

Durch die Amplitudenschwellwertfunktion bzw. die Wahl von mehreren Amplitudenschwellwertfunktionen kann eine Aussage zur Partikelgröße bzw. zur Partikelgrößenverteilung getroffen werden, wobei die Rückwandechohöhe und -form zur Kalibrierung eingesetzt werden kann. Durch letzteres ist dann auch eine Aussage zur absoluten Partikelgröße bzw. Partikelgrößenverteilung möglich. Andernfalls erhält man eine qualitative Aussage. Die Amplitudenschwellwertfunktion kann auch mathematisch an das Rückwandecho gekoppelt werden um Einkoppelschwankungen vom Einkoppel- bzw. Empfangsmedium ins fließende Medium zu korrigieren.

Die Amplitudenschwellwertfunktion hat vorzugsweise einen konstanten zeitlichen Verlauf oder, um beispielsweise die akustische Dämpfung im Messmedium zu korrigieren, einen logarithmischen oder exponentiellen Verlauf. Die akustische Dämpfung folgt z.B. einer Exponentialfunktion mit negativem Exponenten. Durch Multiplikation mit einer Exponentialfunktion mit positivem Exponenten, lässt sich der Dämpfungseinfluss korrigieren.

Die Aufbringung eines Benetzungsschuhs (12), siehe Figur 5, ermöglicht die lokal steuerbare Benetzung des Wellenleiters mit dem Medium. Hierbei wird eine sich im fließenden Medium auflösende Hülle (11), in der sich eine die Benetzung hervorrufende Substanz (10) befindet, am Ende des Wellenleiters 2 platziert. Nach Eintauchen in das Medium 3 löst sich der Benetzungsschuh (12) auf und die Benetzung hervorrufende Substanz (10) wird somit lokal freigesetzt. Eine weitere Möglichkeit besteht darin, die Benetzung hervorrufende Substanz zu schmelzen und den Wellenleiter (2) (8) mit einem Ende in eine flüssige, die Benetzung hervorrufende Substanz (10) einzutauchen.

Als die eine Benetzung hervorrufende Substanz (10) kommen bevorzugt für eine Metallschmelze und insbesondere Aluminiumschmelze gängige Schmelzsalze zum Einsatz (Salz1: ungefähre Zusammensetzung: KCl (47,6 %), NaCl (45,7 %), SO4 (2,14 %), CaF2 (0,14 %); Salz 2: ungefähre Zusammensetzung: KCl (50 %), NaCl (50 %)).

Beispielsweise können die Salze in eine Hülle aus Aluminiumfolie eingefüllt werden, die als äußere Schicht (11) dient. Die Hülle wird anschließend über die Enden der Wellenleiter gestülpt (siehe Fig. 5) und löst sich in der Flüssigkeit/Metallschmelze auf.

Auch kann die Hülle aus einem Material bestehen, welches sich in der Flüssigkeit schmilzt oder löst.

### Bezugszeichenliste

- 1: Ultraschallwandler
- 2: Wellenleiter
- 3: Flüssigkeit, insbesondere Suspension
- 4: Gefäß
- 4a: Begrenzungswand
- 5: Ultraschallreflektor
- 6: Auswerteeinrichtung
- 7: weiterer Ultraschallwandler
- 8: Wellenleiter
- 9: Fokus Ultraschallfeld
- 10: äußere Schicht
- 11: Hülle
- 12: Benetzungsschuh
- 13: Fokus Ultraschallfeld

## Patentansprüche

1. Verfahren zur quantitativen Bestimmung von Anzahl und Größe von in einem Gefäß (4) fließenden Medium, nämlich in Form einer Metallschmelze, enthaltenen partikulären Komponenten, bei dem Ultraschallwellen in das fließende Medium einkoppeln, die zumindest teilweise an den partikulären Komponenten reflektieren und deren reflektierte Ultraschallwellenanteile in Form von Ultraschallzeitsignalen detektiert werden, die der quantitativen Bestimmung zugrunde gelegt werden, **gekennzeichnet durch** folgende Verfahrensschritte:
- Einkoppeln der Ultraschallwellen in das fließende Medium mit einem an einen Ultraschallwandler akustisch gekoppelten Wellenleiter, der zumindest abschnittsweise in das fließende Medium eintaucht, derart, dass zumindest ein Teil der eingekoppelten Ultraschallwellen einer Reflexion an einem das fließende Medium begrenzenden Wandbereich (4a) des Gefäßes (4) oder eines innerhalb des Gefäßes (4) eingebrachten Reflektors (5) unterliegen, durch die ein dem Wandbereich (4a) oder dem Reflektor (5) zuordenbares Echo-Ultraschallzeitsignal erzeugt wird, wobei das Einkoppeln der Ultraschallwellen in das innerhalb des Gefäßes (4) fließende Medium mit einer quer zur Strömungsrichtung des fließenden Mediums orientierten Hauptausbreitungsrichtung derart fokussiert erfolgt, so dass die Ultraschallwellen in einem längs der Hauptausbreitungsrichtung befindlichen Fokuspunkt fokussiert werden, der in Hauptausbreitungsrichtung vor oder nach dem Wandbereich (4a) des Gefäßes (4) oder dem Reflektor (5) liegt, an dem sich die längs der Hauptausbreitungsrichtung ausbreitenden Ultraschallwellen orthogonal oder geneigt auftreffen und reflektiert werden,
- Detektieren der reflektierten Ultraschallwellenanteile im Bereich oder am Ort des Einkoppelns,
- Kalibrieren der Ultraschallzeitsignale auf Grundlage des jeweils in den Ultraschallzeitsignalen enthaltenen Echo-Ultraschallzeitsignals,
wobei die Kalibrierung das Ermitteln wenigstens einer Amplitudenschwellwertfunktion umfasst, die zu jedem detektierten Ultraschallzeitsignal einen Amplitudenschwellwert festlegt, unter Berücksichtigung der Einkoppelbedingungen der Ultraschallwellen in das fließende Medium mittels wenigstens des Echo-Ultraschallzeitsignals,
- Erfassen von den einzelnen Ultraschallzeitsignalen zugeordneten Amplitudenwerten, die jeweils größer als ein zu den jeweiligen Ultraschallzeitsignalen festgelegter Amplitudenschwellwert sind, und
- Zuordnen der erfassten Amplitudenwerte zu Werten, die die Größe und Anzahl der partikulären Komponenten beschreiben.

2. Verfahren nach Anspruch 1,
**dadurch gekennzeichnet, dass** zum Ermitteln der Amplitudenschwellwertfunktion wenigstens eine der nachfolgenden physikalischen Eigenschaften berücksichtigt wird:
- Berücksichtigen der Ultraschallfeldverteilung im fließenden Medium,
- Berücksichtigen der akustischen Dämpfung der Ultraschallwellen im fließenden Medium.

3. Verfahren nach Anspruch 1 oder 2,
**dadurch gekennzeichnet, dass** das Erfassen der Amplitudenwerte, die jeweils größer als ein zum jeweiligen Ultraschallzeitsignal festgelegter Amplitudenschwellwert sind, innerhalb eines festzulegenden Auswertezeitbereiches erfolgt, der einem räumlichen Messbereich innerhalb des fließenden Mediums längs der Hauptausbreitungsrichtung entspricht und zwischen dem Ort des Einkoppelns und dem Wandbereich (4a) des Gefäßes (4) oder dem Reflektor (5) liegt.

4. Verfahren nach einem der Ansprüche 1 bis 3,
**dadurch gekennzeichnet, dass** das Zuordnen der erfassten Amplitudenwerte zu Werten, die die Anzahl der partikulären Komponenten innerhalb des fließenden Mediums beschreiben, auf einer Anzahl oder statistischen Häufigkeit basiert, mit der die pro Ultraschallzeitsignal erfassten Amplitudenwerte über einem jeweils durch die Amplitudenschwellwertfunktion für ein Ultraschallzeitsignal festgelegten Amplitudenschwellwert liegen.

5. Verfahren nach einem der Ansprüche 1 bis 4,
**dadurch gekennzeichnet, dass** das Zuordnen der erfassten Amplitudenwerte zu Werten, die die Größe der partikulären Komponenten beschreiben, jeweils auf dem numerischen Betrag des Amplitudenwertes des Ultraschallzeitsignals basiert.

6. Verfahren nach Anspruch 5,
**dadurch gekennzeichnet, dass** zum Erhalt absoluter Größenwerte ein Kalibrierungswert oder eine Kalibrierungsfunktion ermittelt wird, durch Erfassen eines Ultraschallzeitsignals, insbesondere dessen Amplitude oder/und dessen Signalform, das durch Reflexion der Ultraschallwellen an einem bekannten Ultraschallreflektor gewonnen wird.

7. Verfahren nach Anspruch 6,
**dadurch gekennzeichnet, dass** die wenigstens eine Amplitudenschwellwertfunktion unter Nutzung des Kalibrierungswertes oder der Kalibrierungsfunktion ermittelt wird.

8. Vorrichtung zur quantitativen Bestimmung von Anzahl und Größe von in einem Gefäß (4) fließenden Medium enthaltenen partikulären Komponenten, mit einem Ultraschallwandler, der Ultraschallwellen in das fließende Medium einkoppelt, die zumindest teilweise an den partikulären Komponenten reflektieren und deren reflektierte Ultraschallwellenanteile in Form von Ultraschallzeitsignalen detektierbar und der quantitativen Bestimmung zugrundelegbar sind, wobei die Vorrichtung einen Wellenleiter (2) umfasst, der mit dem Ultraschallwandler akustisch gekoppelt ist, und zumindest abschnittsweise in das fließende Medium derart eintaucht, dass der aus einem Wellenleitermaterial bestehende Wellenleiter (2) wenigstens im Bereich, in dem der Wellenleiter (2) in das fließende Medium eintaucht, mit einer äußeren Schicht (10) umgeben ist, so dass die äußere Schicht (10) zwischen dem sonstigen Wellenleitermaterial und dem fließenden Medium angeordnet ist, und die äußere Schicht (10) eine vom sonstigen Wellenleitermaterial abweichende Stoffzusammensetzung besitzt,
**dadurch gekennzeichnet, dass** die Stoffzusammensetzung der äußeren Schicht (10) wenigstens ein die Benetzung des fließenden Mediums an das Wellenleitermaterial hervorrufendes und unterstützendes Schmelzsalz enthält, und
dass das wenigstens eine Schmelzsalz von einer Hülle (11) oder einer Matrix aus in dem fließenden Medium schmelzbaren Material umgeben ist.

9. Vorrichtung nach Anspruch 8,
**dadurch gekennzeichnet, dass** der Wellenleiter (2) einseitig stumpf, spitzzulaufend oder geometrisch bestimmt geformt zur Einkopplung von fokussierten Ultraschallwellen in das fließende Medium endet,
dass der Wellenleiter (2) zumindest abschnittweise endseitig von der äußeren Schicht (10) umgeben ist, deren Stoffzusammensetzung in Abhängigkeit des fließenden Mediums derart gewählt ist, so dass sich die Stoffzusammensetzung in Kontakt mit dem fließenden Medium auflöst.

10. Verwendung der Vorrichtung nach Anspruch 8 oder 9 zur Bestimmung der Konzentration von Fremdkörpern in der Metallschmelze in Form einer Aluminiumschmelze .

## Claims

1. Method for quantitatively determining number and size of particulate components contained by flowing medium, namely in form of a molten metal, in a vessel (4), in which ultrasound waves couple into the flowing medium, which waves are at least partially reflected on the particulate components, and of which the reflected ultrasound wave fractions are detected in form of ultrasonic time signals, which are used as the basis for the quantitative determination, **characterized by** following method steps:
- coupling the ultrasound waves into the flowing medium with a waveguide coupled acoustically to an ultrasonic transducer, at least a portion of which is immersed in the flowing medium, in such manner that at least some of the coupled in ultrasound waves undergo a reflection on a wall region (4a) of the vessel (4) that delimits the flowing medium or of a reflector (5) introduced inside the vessel (4), by which an echo ultrasonic time signal assignable to the wall region (4a) or the reflector (5) is generated, wherein the coupling of the ultrasound waves into the flowing medium inside the vessel (4) takes place in manner focused with a primary direction of propagation orientated transversely to the flow direction of the flowing medium, with the result that the ultrasound waves are focused in a focal point along the primary direction of propagation, which focal point is located before or after the wall region (4a) of the vessel (4) or the reflector (5) in primary direction of propagation, on which the ultrasound waves propagating along the primary direction of propagation are incident and reflected orthogonally or at an angle,
- detecting the reflected ultrasound wave fractions in the region or at the site of the coupling in,
- calibrating the ultrasonic time signals on the basis of the echo ultrasonic time signal contained in each of the ultrasonic time signals,
wherein the calibration includes calculation of at least one amplitude threshold function, which defines an amplitude threshold value for each detected ultrasonic time signal, taking into account the conditions under which the ultrasound waves are coupled into the flowing medium by means of at least the echo ultrasonic time signals,
- capturing amplitude values assigned to the individual ultrasonic time signals, which values are greater in each case than an amplitude threshold fixed for the respective ultrasonic time signals, and
- assigning the captured amplitude values to values that describe the size and number of the particulate components.

2. Method according to Claim 1, **characterized in that** in order to calculate the amplitude threshold function at least one of the following physical properties is considered:
- consideration of the ultrasound field distribution in the flowing medium,
- consideration of the acoustic attenuation of the ultrasound waves in the flowing medium.

3. Method according to Claim 1 or 2, **characterized in that** the capture of the amplitude values, each of which is greater than an amplitude threshold fixed for the respective ultrasonic time signal, takes place within a definable evaluation time range, which corresponds to a spatial measurement region within the flowing medium along the primary direction of propagation, and is located between the coupling in point and the wall region (4a) of the vessel (4) or the reflector (5).

4. Method according to any one of Claims 1 to 3, **characterized in that** the assignment of the captured amplitude values to values that describe the number of particulate components within the flowing medium is based on a number or statistical frequency with which the amplitude values captured for each ultrasonic time signal lie above an amplitude threshold value fixed for an ultrasonic time signal in each case by the amplitude threshold function.

5. Method according to any one of Claims 1 to 4, **characterized in that** the assignment of the captured amplitude values to values which describe the size of the particulate components, is based in each case on the numeric sum of the amplitude value of the ultrasonic time signal.

6. Method according to Claim 5, **characterized in that** in order to obtain absolute size values, a calibration value or calibration function is calculated by capturing an ultrasonic time signal, in particular the amplitude and/or signal shape thereof, which is obtained via reflection of the ultrasound waves on a known ultrasound reflector.

7. Method according to Claim 6, **characterized in that** the at least one amplitude threshold function is calculated using the calibration value or the calibration function.

8. Device for quantitatively determining number and size of particulate components contained by flowing medium in a vessel (4), with an ultrasonic transducer which couples ultrasound waves into the flowing, which waves are at least partly reflected on the particulate components and the reflected ultrasound wave fractions thereof are detectable in form of ultrasonic time signals and can be used as the basis for the quantitative determination, wherein the device comprises a waveguide (2) that is coupled acoustically to the ultrasonic transducer, and at least a portion of which is immersed in the flowing medium, in such manner that, at least in the region in which the waveguide (2) is immersed in the flowing medium, the waveguide (2) consisting of a waveguide material is surrounded by an outer layer (10), so that the outer layer (10) is arranged between the other waveguide material and the flowing medium, and the outer layer (10) has a material composition that differs from the other waveguide material, **characterized in that** the material composition of the outer layer (10) contains at least one smelting salt which induces and supports the wetting of the waveguide material with the flowing medium, and that the at least one smelting salt is enclosed by a mantle (11) or matrix of a material that is meltable in the flowing medium.

9. Device according to Claim 8, **characterized in that** the waveguide (2) ends bluntly on one side, and is tapered or geometrically determined for coupling focused ultrasound waves into the flowing medium, that the end of the waveguide (2) is at least partially surrounded by the outer layer (10), whose material composition is selected depending on the flowing medium in such manner that the material composition dissolved in contact with the flowing medium.

10. Use of the device according to Claim 8 or 9 for the determination of the concentration of foreign bodies in the molten metal in form of an aluminium melt.

## Revendications

1. Procédé pour la détermination quantitative du nombre et de la taille des composants particulaires contenus dans un milieu en écoulement d'un récipient (4), à savoir sous la forme d'un métal en fusion, dans lequel des ondes ultrasonores sont couplées dans le milieu en écoulement, qui se réfléchissent au moins partiellement sur le composants particulaires et dont les parties d'ondes ultrasonores réfléchies sont détectées sous forme de signaux temporels ultrasonores, qui sont utilisés comme base pour la détermination quantitative, **caractérisé par** les étapes de processus suivantes :
- couplage des ondes ultrasonores dans le fluide en écoulement avec un guide d'ondes couplé acoustiquement à un transducteur à ultrasons, qui est immergé au moins par endroits dans le milieu en écoulement, de telle sorte qu'au moins une partie des ondes ultrasonores couplées soient soumises à une réflexion sur une zone de paroi (4a) du récipient (4) délimitant le fluide en écoulement ou sur un réflecteur (5) disposé à l'intérieur du récipient (4), à travers lesquels un signal temporel ultrasonore d'écho pouvant être affecté à la zone de paroi (4a) ou au réflecteur (5) est généré, dans lequel le couplage des ondes ultrasonores dans le fluide en écoulement à l'intérieur du récipient (4) focalise avec une direction de propagation principale orientée transversalement à la direction d'écoulement du milieu en écoulement, de telle sorte que les ondes ultrasonores soient focalisées dans un point focal situé le long de la direction de propagation principale, qui dans la direction de propagation principale est situé devant ou derrière la zone de paroi (4a) du récipient (4) ou du réflecteur (5), sur laquelle les ondes ultrasonores se propageant dans la direction principale de propagation butent de manière orthogonale ou inclinée et sont réfléchies,
- détection des composantes d'ondes ultrasonores réfléchies dans la zone ou au point de couplage,
- étalonnage des signaux temporels ultrasonores en fonction du signal temporel ultrasonore d'écho contenu dans les signaux temporels ultrasonores respectifs,
dans lequel l'étalonnage comprend la détermination d'au moins une fonction de valeur de seuil d'amplitude, qui définit une valeur de seuil d'amplitude pour chaque signal temporel ultrasonore détecté, en prenant en compte des conditions de couplage des ondes ultrasonores dans le milieu en écoulement au moyen d'au moins le signal temporel ultrasonore,
- acquisition des valeurs d'amplitude attribuées aux signaux temporels ultrasonores individuels, qui sont chacune supérieures à un pour le seuil d'amplitude fixe respectif des signaux temporels ultrasonores, et
- affectation des valeurs d'amplitude détectées à des valeurs d'amplitude, qui représentent la taille et le nombre de composants particulaires.

2. Procédé selon la revendication 1, **caractérisé en ce qu'**au moins une des propriétés physiques suivantes est prise en compte pour déterminer la fonction de valeur de seuil d'amplitude :
- prise en compte de la distribution du champ ultrasonore dans le milieu en écoulement,
- prise en compte de l'amortissement acoustique des ondes ultrasonores dans le milieu en écoulement.

3. Procédé selon la revendication 1 ou 2, **caractérisé en ce que** la détection des valeurs d'amplitude qui sont chacune supérieures à une valeur de seuil d'amplitude spécifiée pour le signal temporel ultrasonore respectif, a lieu dans une plage de temps d'évaluation à définir, qui correspond à une plage de mesure spatiale dans le milieu en écoulement le long de la direction de propagation principale et se situe entre le point de couplage et la zone de paroi (4a) du récipient (4) ou le réflecteur (5).

4. Procédé selon une quelconque des revendications 1 à 3, **caractérisé en ce que** l'affectation des valeurs d'amplitude détectées à des valeurs, qui décrivent le nombre de composants particulaires dans le milieu en écoulement est basée sur un nombre ou une fréquence statistique, avec laquelle les valeurs d'amplitude enregistrées par signal temporel ultrasonore se situent à respectivement une valeur de seuil d'amplitude définie par la fonction de seuil d'amplitude pour un signal temporel ultrasonore.

5. Procédé selon une quelconque des revendications 1 à 4, **caractérisé en ce que** l'attribution des valeurs d'amplitude détectées à des valeurs qui décrivent la taille des composants particulaires est basée dans chaque cas sur la quantité numérique de la valeur d'amplitude du signal temporel ultrasonore.

6. Procédé selon la revendication 5, **caractérisé en ce que** pour obtenir des valeurs absolues supérieures, une valeur d'étalonnage ou une fonction d'étalonnage est déterminée en détectant un signal temporel ultrasonore, en particulier son amplitude et/ou sa forme de signal, qui est obtenu en réfléchissant les ondes ultrasonores sur un réflecteur ultrasonore connu.

7. Procédé selon la revendication 6, **caractérisé en ce que** la au moins une fonction de valeur de seuil d'amplitude est déterminée à partir de la valeur d'étalonnage ou de la fonction d'étalonnage.

8. Dispositif pour la détermination quantitative du nombre et de la taille des composants particulaires contenus dans un fluide en écoulement dans un récipient (4), comportant un transducteur à ultrasons, qui couple des ondes ultrasonores dans le fluide en écoulement, qui se reflètent au moins partiellement sur les composants particulaires et dont les parties d'ondes ultrasonores réfléchies peuvent être détectées sous la forme de signaux temporels ultrasonores et peuvent être utilisées comme base à la détermination quantitative, dans lequel le dispositif comprend un guide d'ondes (2) qui est couplé acoustiquement au transducteur à ultrasons, et est immergé au moins par endroits dans le fluide en écoulement de telle sorte que le guide d'ondes (2), qui se compose d'un matériau de guide d'ondes, soit entouré d'une couche extérieure (10) au moins dans la zone dans laquelle le guide d'ondes (2) est immergé dans le milieu en écoulement, de sorte que la couche extérieure (10) soit disposée entre le reste du matériau de guide d'ondes et le milieu en écoulement, et la couche extérieure (10) possède une composition de substance s'écartant du reste du matériau de guide d'ondes, **caractérisé en ce que** la composition de matériau de la couche extérieure (10) contient au moins un sel fondu qui provoque et supporte le mouillage du milieu en écoulement vers le matériau de guide d'ondes, et **en ce que** le au moins un sel fondu est entouré par une enveloppe (11) ou une matrice de matériau qui peut être fondue dans le milieu en écoulement.

9. Dispositif selon la revendication 8, **caractérisé en ce que** le guide d'ondes (2) se termine émoussé d'un côté, effilé en pointe ou conformé d'une sorte géométriquement déterminée pour le couplage des ondes ultrasonores focalisées dans le milieu en écoulement, que le guide d'ondes (2) est entouré au moins par endroits par la couche extérieure (10), dont la composition de matière est choisie en fonction du milieu en écoulement de sorte que la composition de matière se dissolve au contact du milieu en écoulement.

10. Utilisation du dispositif selon la revendication 8 ou 9 pour déterminer la concentration de corps étrangers dans le métal en fusion sous forme d'une fusion d'aluminium.
